Europäisches Patentamt

European Patent Office

Office européen des brevets

(19)

(11) Numéro de publication: **0 235 014 B1**

(12) **FASCICULE DE BREVET EUROPEEN**

(45) Date de publication de fascicule du brevet: 31.07.91

(51) Int. Cl.⁵: **A61F 13/15**

(21) Numéro de dépôt: 87400252.0

(22) Date de dépôt: 04.02.87

(54) **Change comportant des moyens de fixation du type à boucles et à griffes.**

(30) Priorité: 21.02.86 FR 8602403

(43) Date de publication de la demande: 02.09.87 Bulletin 87/36

(45) Mention de la délivrance du brevet: 31.07.91 Bulletin 91/31

(84) Etats contractants désignés: AT BE CH DE ES FR GB GR IT LI LU NL SE

(56) Documents cités:
EP-A- 0 080 647
WO-A-85/03205
FR-A- 2 586 558
US-A- 3 110 312

(73) Titulaire: **KAYSERSBERG SA**
**Route de Lapoutroie**
**F-68240 Kaysersberg(FR)**

(72) Inventeur: **Pigneul, Raymond**
**2, rue des Vosges**
**F-68320 Durrenentzen(FR)**
Inventeur: **Ruppel, Rémy**
**7, rue des Sorbiers**
**F-68000 Horbourg(FR)**
Inventeur: **Brellmann, Jean**
**5, Petite Rue des Tanneurs**
**F-68000 Colmar(FR)**

(74) Mandataire: **David, Daniel et al**
**Kaysersberg Service Propriété Industrielle**
**54, avenue Hoche**
**F-75360 Paris Cédex 08(FR)**

## Description

L'invention concerne un change complet à usage unique comportant des moyens de fixation du type à boucles et à griffes également connus sous le nom VELCRO (marque commerciale).

Les changes complets à jeter après usage, pour bébés ou adultes incontinents, sont généralement formés d'une masse absorbante constituée d'un matelas de fibres cellulosiques, obtenu par exemple par défibrage à sec de pâte à papier ; le matériau absorbant est placé entre une feuille support généralement imperméable et une feuille perméable aux liquides destinée à venir au contact de la peau de l'utilisateur. Dans le cas où la feuille-support est elle-même perméable, l'étanchéité est obtenue par l'incorporation d'une feuille imperméable aux liquides entre la masse absorbante et la feuille-support.

Le maintien du change autour de la taille du bébé ou de l'adulte est assuré généralement par des attaches adhésives latérales qui sont constituées d'un ruban ou languette de forme rectangulaire dont une section d'extrémité est fixée solidement à la feuille support sur la partie arrière supérieure du change, et la section d'extrémité libre est enduite d'une substance adhésive sensible à la pression, protégée par un papier siliconé.

Au moment de la mise en place du change et de sa fixation autour de la taille, on sépare la partie adhésivée de son papier de protection et on lie la partie arrière à la partie avant, de chaque côté, en appliquant la surface adhésivée des attaches sur la feuille-support dans la région ventrale.

Ce mode de fixation est de réalisation simple et économique, et est relativement fiable. il présente cependant quelques inconvénients :

- Si la surface adhésivée est souillée par une matière étrangère, elle perd partiellement ou totalement son pouvoir adhésif, avec pour conséquence une moindre résistance de la fixation aux contraintes auxquelles elle est soumise pendant le port du change. Pour éviter cela, la mère par exemple devra veiller à maintenir la surface adhésivée bien nette une fois la feuille de protection otée, elle doit faire en sorte de ne pas appliquer les doigts sur cette surface ou bien que du talc ou un corps gras ne vienne s'y déposer. Ceci n'est pas aisé quand il s'agit de maintenir en même temps un bébé.
- Les adhésifs actuels n'offrent qu'un nombre réduit de possibilités de défaire momentanément la fixation, pour déplacer l'attache par exemple dans le but d'ajuster le change ou bien de vérifier son état.
- Suivant les conditions de stockage, la substance adhésive peut s'altérer.

On connaît par EP-A-80647 un change complet à usage unique dont les moyens de fixation autour de la taille de l'utilisateur sont constitués par des attaches adhésives. Celles-ci sont susceptibles d'être appliquées sur une zone de la face avant de la feuille imperméable qui a été renforcée de manière à permettre leur collage et décollage répétés. Cette solution ne résout pas cependant le problème de la perte de pouvoir adhésif en cas de souillure.

On a proposé d'autres modes de fixation ne faisant pas appel à une substance adhésive. Ainsi la demande de brevet FR 8401333 et son certificat d'addition FR 8416844 - correspondant à WO 85/03205 - préconisent l'utilisation de moyens de fixation du type connu sous le nom VELCRO (marque commerciale) et constitués de deux éléments complémentaires, c'est-à-dire, un élément tapissé d'une pluralité de boucles entre lesquelles viennent s'agripper les extrémités de poils, pourvues d'aspérités en forme de crochets ou de griffes, dont est couvert le deuxième élément.

Selon ce brevet, les bords opposés d'un même côté du matelas du change sont pourvus d'un pavé à boucles ou à griffes et leur liaison est assurée par une demi-ceinture, en tissu à boucles ou à griffes complémentaire des pavés précédents. Cette solution élimine les inconvénients cités pour les attaches adhésivées. On peut lier et délier les attaches à volonté, sans crainte d'altérer leurs propriétés d'adhérence ; elles ne risquent par ailleurs aucune détérioration par des matières étrangères. Ce mode de fixation présente toutefois le désavantage d'être plus onéreux, ce qui limite son développement.

On connaît également par les brevets ou demandes de brevet, GB 1428572, US 3081772, US 3141461, US 4402690, US 3359980, WO 83/03754, des vêtements pour bébés, des langes, tous en tissu - qui ne sont donc pas destinés à être jetés après le premier usage - pour lesquels on a prévu des moyens de fixation du type VELCRO.

Des pavés sont cousus sur la face avant du vêtement, et des attaches latérales en matériau complémentaire sont cousues, par une extrémité, à chacun des panneaux latéraux, ou bien sont fixées sur la face interne de la partie arrière. De telles solutions ne sont pas transposables simplement aux articles à usage unique, ne serait-ce que par le mode de fabrication dont le coût doit être pour ces derniers, aussi faible que possible.

L'invention a pour objet un change complet à usage unique, comportant un matelas absorbant de forme allongée constitué par une masse absorbant les liquides comprise entre une feuille-support extérieure et une feuille perméable aux liquides destinée à venir au contact de la peau, les moyens de fixation pour le maintien du matelas autour de la taille de l'utilisateur étant du type à deux éléments à boucles et à griffes coopérant l'un avec l'autre, un premier élément à boucles étant solidaire de le feuille-support près d'une extrémité longitudinale du matelas, et un deuxième élément à griffes étant relié à l'extrémité opposée de la feuille-support, Par rapport à un tel change de l'art antérieur décrit par exemple dans WO 8503205 le change est caractérisé en ce que ledit deuxième élément est fixé par collage ou soudage sur la section d'extrémité libre d'une pièce en matériau souple et résistant formant languette dont l'autre extrémité est fixée de façon permanente, notamment par collage, à le feuille-support.

De préférence, le premier élément à boucles est un pavé de tissu à mailles grattées,

L'avantage d'une telle disposition est de permettre un montage simple et efficace du second élément avec des moyens déjà connus pour la réalisation des attaches adhésivées. En particulier, l'élément peut être fixé par simple collage, au moyen d'une colle thermofusible par exemple.

Un autre avantage réside dans l'importante économie de matière qu'elle autorise. En effet, la taille de l'élément à griffes de l'attache peut être circonscrite à celle strictement nécessaire pour assurer le fonction de fixation.

En choisissant une languette en matériau souple mais peu déformable, on garantit une répartition uniforme des efforts de cisaillement sur la liaison entre l'élément et la languette, limitant ainsi les risques de décollement de l'élement. Ce résultat ne pourrait être obtenu si on fixait cet élément, de faible dimension, directement sur la feuille-support extérieure, en polyéthylène généralement, qui est souple mais peu résistante, les efforts de cisaillement entraînent alors une déformation du support qui conduit à un relâchement progressif de la liaison.

Par ailleurs, avec la solution de l'invention, on peut choisir une zone de liaison attache-feuille extérieure aussi large qu'il est nécessaire pour éviter le fluage du matériau de la feuille, sans avoir à augmenter la taille de l'élément.

L'invention sera mieux comprise, et d'autres avantages apparaîtront à la lecture de la description d'un mode de réalisation qui suit, accompagnée des dessins où :

- la figure 1 représente un change complet à usage unique selon l'invention
- la figure 2 est une vue agrandie d'une des attaches
- la figure 3 est une vue en coupe selon III-III de la figure 2
- la figure 4 est un schéma partiel d'un dispositif de montage des attaches sur les changes.

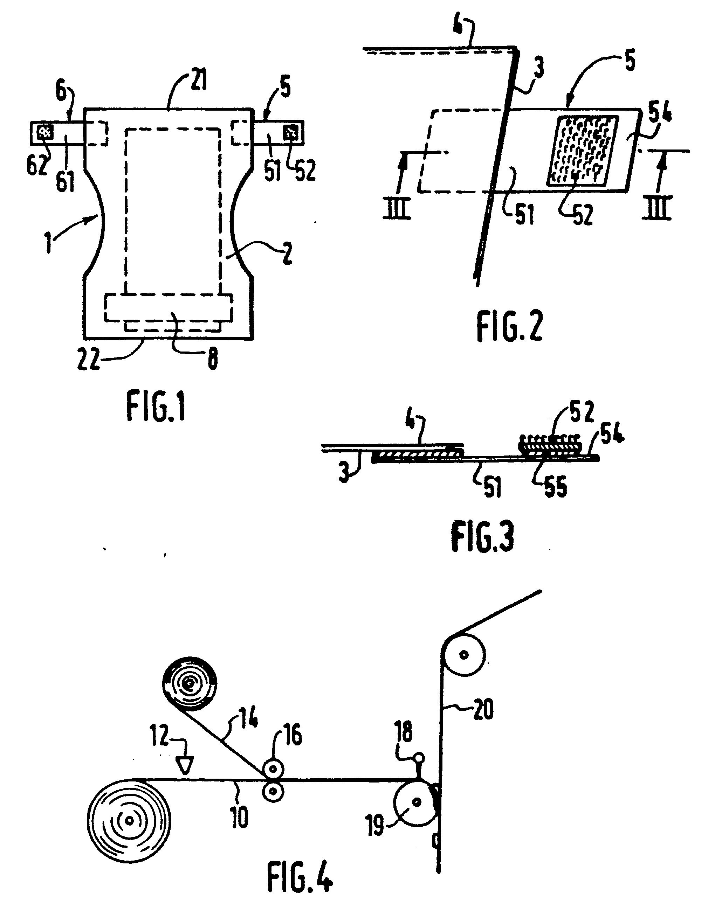

Sur la figure 1 on a représenté un change complet à usage unique désigné par la référence 1 ; il est vu du côté destiné à venir au contact de la peau de l'utilisateur. Il comporte un matelas absorbant 2 de forme sensiblement rectangulaire, constitué d'un matériau absorbant en fibres cellulosiques, par exemple obtenu par défibrage à sec de pâte à papier, enfermé entre une feuille-support extérieure 3 et une feuille perméable aux liquides 4 destinée à venir au contact de la peau. Les matériaux sont généralement un polyéthylène pour la feuille-support, et un non-tissé obtenu par filature directe pour la feuille 4, mais d'autres matériaux sont également utilisés. La feuille-support est le plus souvent imperméable, mais elle peut aussi présenter une certaine perméabilité et être associée à une feuille supplémentaire imperméable disposée entre elle-même et le matériau absorbant. Les deux feuilles sont liées entre elles de manière à former une poche fermée. Le matelas peut comporter des découpes arrondies correspondant au contour des cuisses de l'utilisateur, et être pourvu dans cette zone, de part et d'autre de la masse absorbante, de brins élastiques ou d'autres moyens permettant une bonne adaptation à l'anatomie de l'utilisateur, et d'éviter ainsi les fuites.

Le matelas comporte des attaches latérales, 5 et 6, à proximité du bord 21. Ces attaches comprennent une languette 51, 61 de forme rectangulaire, liée au matelas par une section d'extrémité, et réalisée dans un matériau souple et peu déformable. Cela peut être un polyéthylène, un polypropylène, un polyester, un papier, un non-tissé ou bien une structure stratifiée composée de deux ou plusieurs couches des matériaux précédents. Il peut s'agir par exemple d'un non-tissé fabriqué par filature directe de polyester associé à un film de polyester.

Conformément à l'invention, l'attache comporte également, un élément de fixation du type VELCRO (marque commerciale) 52, 62 rapporté sur la face de la languette située du côté de la feuille 4, à proximité de son bord libre. Cet élément est lié préférentiellement par collage, mais il peut être aussi soudé.

A proximité du bord 22 opposé au bord 21, le matelas comprend au moins un élément de fixation du type VELCRO 8 (marque commerciale), complémentaire des éléments montés sur les attaches. Cet élément est lié par collage ou soudage à la feuille-support. De préférence, les éléments 52, 62 des attaches sont du type à griffes, et l'élément 8 est du type à boucles par exemple un tissu à mailles grattées.

Selon le mode de réalisation représenté, l'élément 8 est rectangulaire et s'étend sur une partie substantielle de la largeur du matelas afin de donner une marge de réglage suffisante lors de la mise en place du change. Toutefois, d'autres réalisations sont envisageables, par exemple deux ou plusieurs éléments en forme de pavés peuvent être disposés judicieusement sur cette face avant pour permettre un réglage optimum des attaches 5 et 6.

L'attache 5 est représentée plus en détail sur les figures 2 et 3. La languette 51 est liée à la feuille-support, le long d'une section d'extrémité, par l'intermédiaire d'une couche continue ou discontinue d'une substance adhésive qui est de préférence de la colle thermofusible. L'élément 52 à griffes est fixé à la languette par l'intermédiaire d'une couche continue ou discontinue d'une substance adhésive 55 qui peut être la même que la précédente. L'élément 52 est fixé légèrement en retrait du bord libre de la languette 51 pour ménager une bande de préhension 54. Cette bande peut être obtenue par repli de l'extrémité de la languette sur elle-même, et former ainsi un bourrelet facilitant la saisie de l'attache.

L'attache 6 est réalisée de la même manière que l'attache 5. Elle comprend une languette 61, un élément à griffes 62 assujetti à la languette par une couche d'adhésif 65, et une bande de préhension 64.

Dans une variante non représentée, l'extrémité de l'attache peut être non pas appliquée sur la face externe de la feuille-support, mais disposée entre la feuille-support et la feuille perméable, de façon que la liaison de l'attache au matelas offre une plus grande résistance à l'arrachement.

On a représenté sur la figure 4, l'exemple d'un dispositif permettant la réalisation des attaches selon l'invention et leur liaison au matelas.

Un ruban 10 est déroulé à partir d'une bobine montée sur un dévidoir, sa largeur est égale à la longueur de la languette des attaches. Un pistolet 12 comportant deux buses de largeur définie dépose la colle thermofusible suivant deux bandes parallèles. Il est évidemment possible de déposer la colle suivant une seule bande. Ensuite, le ruban 14 en matériau constituant l'élément à griffes, est déroulé depuis une bobine montée sur un deuxième dévidoir, et déposé sur la partie adhésivée du ruban 10 prévue à cet effet, avec application d'une pression par passage entre les rouleaux 16.

Le double ruban parvient ensuite à un poste de coupe et transfert qui comprend, de façon connue en soi, un couteau 18 coopérant avec un tambour 19 partiellement perforé dont le volume intérieur est maintenu en dépression. Ce tambour entraîne, à intervalles réguliers, une attache coupée par le couteau 18 et va la déposer sur le film 20 de polyéthylène constituant la feuille-support des changes, où elle se fixe par l'intermédiaire de la couche d'adhésif.

Dans la variante où l'attache est liée entre les deux feuilles de l'enveloppe, le film 20 sur lequel est déposé l'attache peut être le film en matériau perméable ou le film pour le support, et l'enduction de matière adhésive peut être réalisée directement sur l'un des deux films.

## Revendications

1. Change complet à usage unique, comportant un matelas absorbant de forme allongée constitué par une masse absorbant les liquides comprise entre une feuille-support extérieure et une feuille perméable aux liquides destinée à venir au contact de la peau, les moyens de fixation pour le maintien du matelas autour de la taille de l'utilisateur étant du type à deux éléments à boucles et à griffes coopérant l'un avec l'autre, un premier élément (8) à boucles étant solidaire de la feuille-support près d'une extrémité longitudinale (22) du matelas, et un deuxième élément à griffes relié à l'extrémité opposée de la feuille-support, caractérisé en ce que ledit deuxième élément (52, 62) est fixé par collage ou soudage sur la section d'extrémité libre d'une languette (51, 61) dont l'autre extrémité est fixée de façon permanente, notamment par collage, à la feuille-support.

2. Change selon la revendication 1, caractérisé an ce que le second élément (52, 62) est disposé en retrait par rapport au bord de l'extrémité libre de la languette de manière à ménager une bande de préhension (54, 64).

3. Change selon l'une des revendications précédentes, caractérisé en ce que la languette (51) de l'attache est en papier.

4. Change selon l'une des revendications 1 et 2, caractérisé en ce que la languette (51) est en un matériau tel que le polyéthylène, le polyester, le polypropylène.

5. Change selon l'une des revendications 1 et 2, caractérisé en ce que la languette (51) est en matériau non-tissé.

6. Change selon l'une des revendications 1 et 2, caractérisé en ce que la languette (51) est un complexe stratifié comportant deux ou plusieurs matériaux selon les revendications 4 à 5.

7. Change selon l'une des revendications précédentes, caractérisé en ce que la languette est fixée au matelas entre la feuille perméable et la feuille-support.

## Claims

1. A disposable napkin, comprising an absorbent cushion of elongate shape formed by a fluid absorbing mass contained between an external support sheet and a sheet which can be permeated by fluids designed to come into contact with the skin, the fastening means for holding the cushion around the user's waist being of the type having two members with loops and hooks engaging with one another, a first looped member (8) being rigid with the support sheet close to one longitudinal end (22) of the cushion and a second hooked member being joined to the opposite end of the support sheet, characterized in that the second member (52, 62) is fastened by gluing or fusion to the free end section of a tongue (51, 61) whose other end is permanently fastened, in particular by gluing, to the support sheet.

2. A napkin as claimed in claim 1, characterized in that the second member (52, 62) is disposed to the rear of the edge of the free end of the tongue so as to provide a handling strip (54, 64).

3. A napkin as claimed in one of the preceding claims, characterized in that the tongue (51) of the fastener is of paper.

4. A napkin as claimed in one of claims 1 and 2, characterized in that the tongue (51) is of a material such as polyethylene, polyester or polypropylene.

5. A napkin as claimed in one of claims 1 and 2, characterized in that the tongue (51) is of non-woven material.

6. A napkin as claimed in one of claims 1 and 2, characterized in that the tongue (51) is a layered composite comprising one or a plurality of materials as claimed in claims 4 and 5.

7. A napkin as claimed in one of the preceding claims, characterized in that the tongue is fastened to the cushion between the permeable sheet and the support sheet.

**Patentansprüche**

1. Einweg-Windel mit einer absorbierenden Matte länglicher Form, die aus einer die Flüssigkeiten absorbierenden Masse besteht, welche zwischen einer äußeren Trägerfolie und einer mit der Haut in Berührung kommenden flüssigkeitsdurchlässigen Folie angeordnet ist, wobei die Befestigungsmittel zum Halten der Matte um die Hüften des Benutzers aus zwei zusammenwirkenden Elementen bestehen, wobei ein schlaufenartiges erstes Element (8) als Teil der Trägerfolie nahe an einem Stirnende (22) der Matte vorgesehen ist und ein häkchenartiges zweites Element mit dem entgegengesetzten Ende der Trägerfolie verbunden ist, dadurch gekennzeichnet, daß das zweite Element (52, 62) durch Kleben oder Schweißen an dem freien Endabschnitt einer Lasche (51, 61) befestigt ist, dessen anderes Ende permanent, insbesondere durch Kleben, an der Trägerfolie befestigt ist.

2. Windel nach Anspruch 1, dadurch gekennzeichnet, daß das zweite Element (52, 62) bezüglich des Randes des freien Endes der Lasche zurückversetzt angeordnet ist, so daß ein Griffband (54, 64) gebildet wird.

3. Windel nach einem der vorhergehenden Ansprüche, dadurch gekennzeichnet, daß die Befestigungslasche (51) aus Papier besteht.

4. Windel nach einem der Ansprüche 1 und 2, dadurch gekennzeichnet, daß die Lasche (51) aus einem Material wie Polyäthylen, Polyester, Polypropylen besteht.

5. Windel nach einem der Ansprüche 1 und 2, dadurch gekennzeichnet, daß die Lasche (51) aus einem Vliesstoff besteht.

6. Windel nach einem der Ansprüche 1 und 2, dadurch gekennzeichnet, daß die Lasche (51) als mehrlagiges Gebilde mit einem oder mehreren Materialien nach den Ansprüchen 4 bis 5 ausgebildet ist.

7. Windel nach einem der vorhergehenden Ansprüche, dadurch gekennzeichnet, daß die Lasche an der Matte zwischen der durchlässigen Folie und der Trägerfolie befestigt ist.

FIG.1

FIG.2

FIG.3

FIG.4